# EUROPÄISCHE PATENTSCHRIFT

(11) **EP 3 409 300 B1**
(45) Veröffentlichungstag und Bekanntmachung des Hinweises auf die Patenterteilung: **27.11.2019**
(21) Anmeldenummer: 17174022.8
(22) Anmeldetag: 01.06.2017
(51) Int. Cl.: A61L 2/20, A61L 9/015, A61L 2/22

(54) **DEKONTAMINATIONSANORDNUNG, SYSTEM SOWIE DEKONTAMINATIONSVERFAHREN**
DECONTAMINATION ARRANGEMENT, SYSTEM AND DECONTAMINATION METHOD
SYSTÈME DE DÉCONTAMINATION, SYSTÈME ET PROCÉDÉ DE DÉCONTAMINATION

(43) Veröffentlichungstag der Anmeldung: 05.12.2018
(73) Patentinhaber: Metall + Plastic GmbH, 78315 Radolfzell (DE)
(72) Erfinder: KLEINMANN, Stefan, 78315 Radolfzell (DE); VERTONGEN, David, 3000 Leuven (BE); KAßNER, Thomas, 78315 Radolfzell (DE)
(74) Vertreter: Patentanwälte Behrmann Wagner PartG mbB

(56) Entgegenhaltungen:
- EP-A1- 1 764 115
- EP-A1- 2 786 811
- WO-A1-2013/003967
- US-A1- 2013 004 390
- US-B2- 6 840 744

## Beschreibung

Die Erfindung betrifft eine Dekontaminationsanordnung, insbesondere für pharmatechnische Anwendungen, gemäß dem Oberbegriff des Anspruchs 1, umfassend mindestens einen zu dekontaminierenden Raum, insbesondere einen Isolatorraum, bevorzugt ein Manipulatorraum und/oder einen von dem Manipulatorraum über einen Membran getrennten Plenumraum, mit einem Dekontaminationsflüssigkeitsvorratsbehälter aus dem mehrere in dem zu dekontaminierenden Raum angeordnete, bevorzugt als Zweistoffdüsen ausgebildete, Zerstäuber zum Erzeugen eines Dekontaminationsflüssigkeitsaerosols, insbesondere eines Wasserstoffperoxidaerosols, über mittels elektronische Steuermittel ansteuerbare Ventilmittel mit Dekontaminationsflüssigkeit, bevorzugt Wasserstoffperoxid, versorgbar sind, wobei die Zerstäuber fluidleitend über die Ventilmittel mit einer (gemeinsamen) einen Einlass und einen davon separaten Auslass aufweisenden Ringleitung verbunden sind, in deren Einlass Dekontaminationsflüssigkeit aus dem Dekontaminationsflüssigkeitsvorratsbehälter mittels einer Pumpe förderbar ist und aus deren Auslass Dekontaminationsflüssigkeit zurück in den Dekontaminationsflüssigkeitsvorratsbehälter strömen kann, und wobei der Ringleitung Druckeinstellmittel zum Halten des Flüssigkeitsdrucks in der Ringleitung in einem vorgegebenen Flüssigkeitsdruckbereich zugeordnet sind.

Ferner betrifft die Erfindung ein System gemäß Anspruch 10, umfassend einen Isolator, der bevorzugt eingerichtet ist für pharmazeutische Anwendungen, mit einem zu dekontaminierenden Raum, insbesondere einem Manipulatorraum und/oder einem von dem Manipulatorraum über eine Membran zur Laminierung eines Luftstroms eines Umluftgebläses des Isolators getrennten Plenumraum sowie mit einer Dekontaminationsanordnung. Das Umluftgebläse ist bevorzugt in einem Umlufterzeugerraum oberhalb des vorerwähnten Plenumraums angeordnet. Ganz besonders bevorzugt ist der Manipulatorraum über einen, weiter bevorzugt zwischen zwei transparenten Scheiben ausbildenden Rückströmkanal luftleitend mit dem Umlufterzeugerraum verbunden. Bevorzugt ist zur Realisierung des Merkmals der Einrichtung des Isolators für pharmatechnische Anwendungen in dem Manipulatorraum eine Dosier- und/oder eine Abfülleinrichtung für einen pharmazeutischen Wirkstoff und/oder eine Herstellungseinrichtung für einen solchen Wirkstoff angeordnet. Ganz besonders bevorzugt ist es, wenn der Manipulatorraum über Handschuhe für einen manuellen Eingriff von außen für eine Bedienperson zugänglich ist, wobei die Handschuhe bzw. sogenannte Handschuhports in einer Seitenwand des Manipulatorraums angeordnet bzw. festgelegt sind.

Ferner betrifft die Erfindung ein Dekontaminationsverfahren gemäß dem Oberbegriff des Anspruchs 11, insbesondere zum Betreiben einer erfindungsgemäßen Dekontaminationsanordnung und/oder eines erfindungsgemäßen Systems, wobei ein zu dekontaminierender Raum, insbesondere ein Isolatorraum, bevorzugt ein Manipulatorraum und/oder ein von dem Manipulatorraum über eine Membran getrennter Plenumraum, dekontaminiert wird, indem mittels in dem zu dekontaminierenden Raum angeordnete, bevorzugt als mit Druckluft versorgbare Zweistoffdüsen ausgebildete, Zerstäuber ein Dekontaminationsflüssigkeitsaerosol aus einer Dekontaminationsflüssigkeit, insbesondere Wasserstoffperoxid, erzeugt wird, die mit der Dekontaminationsflüssigkeit aus einem Dekontaminationsflüssigkeitsvorratsbehälter über Ventilmittel versorgt werden, wobei die Zerstäuber fluidleitend über die Ventilmittel mit einer einen Einlass und einen davon separaten Auslass aufweisenden Ringleitung verbunden sind, in deren Einlass Dekontaminationsflüssigkeit aus dem Dekontaminationsflüssigkeitsvorratsbehälter mittels einer Pumpe gefördert wird und aus deren Auslass, insbesondere während einer Abgabe von Dekontaminationsflüssigkeit über die Zerstäuber, Dekontaminationsflüssigkeit zurück in den Dekontaminationsflüssigkeitsvorratsbehälter strömt, und wobei ein Flüssigkeitsdruck der Dekontaminationsflüssigkeit in der Ringleitung über Druckeinstellmittel in einem vorgegebenen Druckbereich gehalten wird, bevorzugt indem ein maximaler Flüssigkeitsdruck begrenzt wird.

Im Rahmen der vorliegenden Offenbarung ist das Merkmal der Versorgung der Zerstäuber über Ventilmittel so zu verstehen, dass die Dekontaminationsflüssigkeit auf ihrem Strömungsweg vom Dekontaminationsflüssigkeitsvorratsbehälter zu den Zerstäubern entsprechende Ventilmittel passieren bzw. durchströmen muss. Dabei sind die Ventilmittel über Steuermittel (Steuereinrichtung) ansteuerbar, insbesondere öffen- und schließbar. Weiter bevorzugt sind die Ventilmittel so ausgebildet, dass mit diesen eine fluidleitende Verbindung zwischen den Zerstäubern und dem Dekontaminationsflüssigkeitsvorratsbehälter, ganz besonders bevorzugt einer später noch zu erläuternden, erfindungsgemäß vorgesehenen Ringleitung durch entsprechende Ansteuerung über die Steuermittel unterbrechbar ist.

In der WO 2011/085735 A1 bzw. der EP 2 719 962 A1 ist eine Dekontaminationsanordnung, umfassend einen pharmatechnischen Isolator sowie einen H₂O₂-Verdampfer beschrieben. Der H₂O₂-Verdampfer (Blitzverdampfer) ist über eine Versorgungsleitung unmittelbar mit einem Plenumraum verbunden, der von einem darunter befindlichen Manipulatorraum über eine Membran zur Erzeugung einer laminaren Luftströmung im Betrieb des Isolators getrennt ist. In einer Dekontaminationsphase wird über die Versorgungsleitung Wasserstoffperoxiddampf in den Plenumraum eingeleitet, wobei der Wasserstoffperoxiddampf durch die Membran nach unten in den Manipulatorraum gelangt.

Aus der US 6,010,400 A ist es bekannt, Luft im Umluftbetrieb durch einen Verdampfer zu fördern, d.h. diesen nicht mit steriler Druckluft von außen zu versorgen, wobei der Wasserstoffperoxiddampf unmittelbar in einen Manipulatorraum eingeleitet wird, unter Umgehung eines katalysatorgetränkten Hochleistungsschwebstofffilters in einem Bereich oberhalb des Manipulatorraums.

Die WO 2013/003967 A1 beschreibt eine Dekontaminationsanordnung, bei der Wasserstoffperoxid nicht klassisch über einen Blitzverdampfer verdampft wird, sondern bei welcher Zweistoffdüsen zum Einsatz kommen, um das flüssige Wasserstoffperoxid zu zerstäuben, also ein Aerosol zu erzeugen. Bei der bekannten Dekontaminationsanordnung ist ein Dekontaminationsflüssigkeitsvorratsbehälter (Reservoir) und der Zerstäuber integraler Bestandteil einer als Ganzes in dem zu dekontaminierenden Raum installierbaren Vorrichtung.

Daneben sind aus der Praxis Lösungen bekannt, bei denen mehrere Zerstäuber an einen gemeinsamen Dekontaminationsflüssigkeitsvorratsbehälter über jeweils eine am zugehörigen Zerstäuber endende Versorgungsleitung angeschlossen sind. Jeder Versorgungsleitung sind dabei eine Pumpe und ein Ventil zugeordnet. Vor Beginn eines Dekontaminationszyklus müssen die einzelnen Versorgungsleitungen über die jeweilige Pumpe befüllt werden; erst danach beginnt der eigentliche Dekontaminationszyklus, bei welchem Dekontaminationsflüssigkeit über die Zerstäuber in den zu dekontaminierenden Raum in Form eines Aerosols abgegeben wird. Die in den Versorgungsleitungen befindliche Luft muss dabei in den zu dekontaminierenden Raum über die Zerstäuber abgegeben werden. Nach Beendigung des Dekontaminationszyklus müssen zur Bestimmung der Menge auf der insgesamt über die Zerstäuber abgegebenen Dekontaminationsflüssigkeit die Versorgungsleitungen wieder in den Dekontaminationsflüssigkeitsvorratsbehälter hinein entleert werden, wozu die Förderpumpen rückwärts betrieben werden. Die Gesamtmenge an verbrauchter bzw. abgegebener Dekontaminationsflüssigkeit wird dabei ermittelt durch Differenzbildung aus dem Vorratsbehältergewicht vor dem Befüllen der Versorgungsleitungen und dem Gewicht des Vorratsbehälters nach erfolgtem Dekontaminationszyklus und nachfolgender Entleerung der Versorgungsleitungen. Nachteilig bei der bekannten Dekontaminationsanordnung bzw. dem bekannten Dekontaminationsverfahren ist die für einen validieren Dekontaminationsprozess notwendige, aufwendige Bestimmung der Gesamtmenge an abgegebener Dekontaminationsflüssigkeit, da hierfür sämtliche Versorgungsleitungen wieder entleert werden müssen. Darüber hinaus ist eine Detektion aufwendig und fehleranfällig, ob tatsächlich vor Beginn eines Dekontaminationszyklus sämtliche Versorgungsleitungen vollständig mit Dekontaminationsflüssigkeit gefüllt sind. Zudem kann es beim Befüllen der Versorgungsleitungen bereits zu einem ungewollten Austritt von Wasserstoffperoxid durch die Zerstäuber vor Beginn des Dekontaminationszyklus kommen. Ferner ist die Bestimmung der Gesamtmenge an abgegebener Dekontaminationsflüssigkeit aufgrund möglicher Dekontaminationsflüssigkeitsreste in den Versorgungsleitungen nach deren Entleerung fehlerbehaftet. Zudem sind die Vor- und Nachbereitungen zum eigentlichen Dekontaminationszyklus zeitintensiv.

Aus der EP 2 786 811 A1 ist eine Vorrichtung zur Versorgung von Verbrauchern mit Reinigungs- und/oder Desinfektionsfluid zum bevorzugten Einsatz in einer Getränkeabfüllanlage bekannt, wobei das Fluid aus einem Reservoir zu den Verbrauchern über eine Ringleitung gefördert wird, über die überschüssiges Fluid zurück in das Reservoir gelangen kann. Für den bestimmungsgemäßen Einsatz der bekannten Vorrichtung ist die Bestimmung der exakten Menge der an die Verbraucher abgegebener Fluidmenge irrelevant.

Aus der US 6,840,744 B2 ist zudem ein System bekannt, welches ein Messmittel zur Bestimmung der Gesamtmenge der von an Zerstäuber während eines Dekontaminationszyklus abgegebener Dekontaminationsflüssigkeit vorgesehen ist. Dabei wird offenbart, dass die Messmittel Wägemittel zum Bestimmen einer Dekontaminationsflüssigkeitsmenge in einem Dekontaminationsflüssigkeitsbehälter umfasst.

Zum weiteren Stand der Technik werden die EP 1 764 115 A1 sowie die US 2013/004390 A1 genannt.

Ausgehend vom vorgenannten Stand der Technik liegt der Erfindung die Aufgabe zugrunde, eine verbesserte Dekontaminationsanordnung, insbesondere für pharmatechnische Anwendungen sowie ein verbessertes Dekontaminationsverfahren anzugeben, welches insbesondere eine vereinfachte und genauere Bestimmung der Gesamtmenge an abgegebener Dekontaminationsflüssigkeit ermöglicht. Bevorzugt soll zudem die Verteilung des Dekontaminationsflüssigkeitsaerosols im zu dekontaminierenden Raum verbessert und/oder beschleunigt werden. Darüber hinaus besteht die Aufgabe darin, ein entsprechend verbessertes Isolatorsystem anzugeben.

Diese Aufgabe wird hinsichtlich der Dekontaminationsanordnung mit den Merkmalen des Anspruchs 1 gelöst.

Hinsichtlich des Dekontaminationsverfahrens wird die Aufgabe mit den Merkmalen des Anspruchs 11 gelöst.

Hinsichtlich des Isolatorsystems wird die Aufgabe mit den Merkmalen des Anspruchs 10 gelöst.

Vorteilhafte Weiterbildungen der Erfindung sind in den Unteransprüchen angegeben. In den Rahmen der Erfindung fallen sämtliche Kombinationen aus zumindest zwei von den in der Beschreibung, den Ansprüchen und/oder den Figuren offenbarten Merkmalen.

Der Erfindung liegt der Gedanke zugrunde, einzelne Zerstäuber nicht mehr wie im Stand der Technik über separate an den jeweiligen Zerstäubern endende Versorgungsleitung mit Dekontaminationsflüssigkeit, insbesondere Wasserstoffperoxid aus einem Dekontaminationsflüssigkeitsvorratsbehälter zu versorgen, sondern mehrere in den zu dekontaminierenden Raum angeordnete Zerstäuber fluidleitend mit einer (gemeinsamen) Ringleitung zu verbinden, also einer Leitung, die aus dem Dekontaminationsflüssigkeitsvorratsbehälter ausmündet und endseitig bzw. auslassseitig wieder in diesen einmündet, sodass Dekontaminationsflüssigkeit aus dem Dekontaminationsflüssigkeitsvorratsbehälter durch die Ringleitung im Kreislauf förderbar ist und somit wieder nach Durchströmen der Ringleitung in den (gemeinsamen) Dekontaminationsflüssigkeitsvorratsbehälter fließt. Gleichzeitig sind der Ringleitung Druckeinstellmittel zugeordnet, mit denen der Flüssigkeitsdruck in der Ringleitung in einem vorgegebenen Flüssigkeitsdruckbereich, insbesondere zwischen 0,05bar und 0,8bar, ganz besonders bevorzugt zwischen 0,1bar und 0,8bar, noch weiter bevorzugt zwischen 0,1bar und 0,5bar, besonders bevorzugt zwischen 0,2bar und 0,5bar haltbar ist. In Abhängigkeit der eingesetzten Zerstäuber kann der Druckbereich auch anders gewählt werden. Bevorzugt liegt in der Ringleitung während der Abgabe von Dekontaminationsflüssigkeit über die Zerstäuber dauerhaft ein Druck über 0 bar an, um sicherzustellen, dass alle Zerstäuber mit ausreichend Dekontaminationsflüssigkeit beaufschlagt werden oder beaufschlagbar sind.

Durch das Vorsehen einer Ringleitung wird der im Stand der Technik vergleichsweise schwierig kontrollierbare Schritt der Leitungsbefüllung vereinfacht, da die Ringleitung dann sicher vollständig mit Dekontaminationsflüssigkeit gefüllt ist, wenn auslassseitig Dekontaminationsflüssigkeit wieder im Dekontaminationsflüssigkeitsvorratsbehälter ankommt. Ein Verdrängen von Luft aus der Ringleitung über die Zerstäuber in den zu dekontaminierenden Raum hinein ist nicht notwendig. Auch können durch das Vorsehen der Ringleitung an allen Zerstäubern im Wesentlichen gleiche Flüssigkeitsdruckverhältnisse realisiert werden. Darüber hinaus ermöglicht es das Vorsehen der Ringleitung, die Gesamtmenge an über die Zerstäuber in den zu dekontaminierenden Raum abgegebener Dekontaminationsflüssigkeitsmenge sehr genau und deutlich einfacher festzustellen als im Stand der Technik, da hierzu eine Entleerung der als Ringleitung ausgebildeten Versorgungsleitung nicht notwendig ist. Vielmehr kann die Differenz gebildet werden zwischen dem Gewicht des Dekontaminationsflüssigkeitsvorratsbehälters bei gefüllter Ringleitung vor dem Beginn eines Dekontaminationszyklus, d.h. vor Beginn des Zerstäubungsprozesses und dem Gewicht des Dekontaminationsflüssigkeitsvorratsbehälters nach Beendigung des Dekontaminationszyklus, d.h. nach Beendigung des Zerstäubungsvorgangs, jedoch wiederum bei (noch) mit Dekontaminationsflüssigkeit gefüllter Ringleitung. Eine Entleerung der Ringleitung zur Bestimmung der abgegebenen Dekontaminationsflüssigkeitsmenge ist nicht mehr notwendig. Es ist jedoch trotzdem denkbar und bevorzugt nach Beendigung eines Dekontaminationszyklus bzw. vor einem erneuten Dekontaminationszyklus die Ringleitung zu entleeren, insbesondere, wie später noch erläutert werden wird mit Druckluft leer zu drücken, um Dekontaminationsflüssigkeitsrückstände über einen längeren Zeitraum in der Ringleitung zu vermeiden - da dieser fakultative Verfahrensschritt jedoch nicht mehr notwendig ist zu Validierung des Prozesses bzw. zur Bestimmung der abgegebenen Dekontaminationsflüssigkeitsmenge sind etwaige Kleinstflüssigkeitsrückstände nach der Entleerung im Hinblick auf die Genauigkeit der bestimmten, abgegebenen Dekontaminationsflüssigkeitsmenge ohne Einfluss.

Die erfindungsgemäß der Ringleitung zugeordneten Druckeinstellmittel dienen dazu, den Flüssigkeitsdruck in der Ringleitung in einem vorgegebenen bzw. definierten Bereich zu halten. Hierdurch werden weitgehend gleiche Strömungs- bzw. Druckverhältnisse an den an die Ringleitung angeschlossenen Zerstäubern gewährleistet. Bevorzugt sind die an die Ringleitung angeschlossenen Zerstäuber gleichweit von der Ringleitung beabstandet, um somit unterschiedliche Leitungslängen zwischen der Ringleitung und den Zerstäubern, zumindest weitgehend, zu verhindern. Zudem ermöglichen die Druckeinstellmittel in Kombination mit der Ringleitung, aufgrund der im Wesentlichen gleichbleibenden Flüssigkeitsdruckverhältnisse an den an die Ringleitung angeschlossenen Zerstäubern die über die Gesamtheit der Zerstäuber und/oder einzelne der Zerstäuber und/oder Gruppen der Zerstäuber in den zu dekontaminierenden Raum abgegebene Dekontaminationsflüssigkeitsmenge einfach über die Öffnungs- und Geschlossenzeit der Ventilmittel vorzugeben. Dies wiederum ermöglich, was in Weiterbildung der Erfindung mit Vorteil vorgesehen ist, die Ventilmittel als Schaltventile auszubilden, die ganz besonders bevorzugt intermittierend (gepulst bzw. getaktet) betrieben werden, wobei die zeitsteuerbare bzw. zeitgesteuerte Offen- und Geschlossenzeit der Schaltventile die jeweils abgegebene Dekontaminationsflüssigkeitsmenge, bei gegebenem Flüssigkeitsdruck in der Ringleitung bestimmt.

Bevorzugt ist der Fördervolumenstrom, insbesondere durch Einstellen oder Vorgabe der Pumpenleistung und Einstellung bzw. Vorgabe der Druckeinstellmittel so bemessen, dass ein größerer Volumenstrom der Ringleitung zugeführt wird als insgesamt, d.h. gleichzeitig über die Zerstäuber abgegeben wird, so dass während des Zerstäubens immer auch noch Dekontaminationsflüssigkeit aus der Ringleitung in den Vorratsbehälter zurückströmt. Dies dient gleichzeitig zur Kontrolle bzw. als Garantie dafür, dass alle an die Ringleitung angeschlossenen Zerstäuber mit Dekontaminationsflüssigkeit versorgt sind oder versorgbar sind.

Über die zwischen den Zerstäubern und der Ringleitung angeordneten Ventilmittel kann also der Zustrom von Dekontaminationsflüssigkeit aus der Ringleitung zu den Zerstäubern beeinflusst werden, insbesondere kann eine fluidleitende Verbindung hergestellt und unterbrochen werden. Wie bereits erwähnt und später noch im Detail erläutert werden wird, umfassen die Ventilmittel bevorzugt eine Mehrzahl von Ventilen, insbesondere diskret schaltende Schaltventilen, bevorzugt in der Form von 2/2-Wegeventil, wobei bevorzugt zwischen einzelnen Zerstäubern, insbesondere sämtlichen Zerstäubern und der zugeordneten Ringleitung jeweils ein solches Ventil angeordnet ist und/oder zwischen der Ringleitung und mindestens einer Gruppe von Zerstäubern. Für den Fall des Vorsehens mehrerer an die Ringleitung angeordneter Zerstäubergruppen ist es bevorzugt, jeder Gruppe ein solches Ventil zuzuordnen. Für eine optimale Steuerung und Beeinflussbarkeit der Dekontaminationsflüssigkeitsaerosolerzeugung es ist jedoch bevorzugt, jedem Zerstäuber ein eigenes Ventil, insbesondere ein Schaltventil zuzuordnen, also ein Ventil, das zwischen einer vollständig geschlossenen und einer vollständig geöffneten Ventilstellung umschaltbar ist.

Besonders bevorzugt ist es, wenn die Ventile möglichst nah an der Ringleitung angeordnet sind, bevorzugt unmittelbar an diese angrenzen.

Bevorzugt ist eine Dekontaminationsflüssigkeitsleitungslänge zwischen mehreren der Zerstäuber, insbesondere sämtlichen Zerstäubern und den diesen jeweils zugeordneten Ventilen (Ventilmittel) und/oder der Ringleitung möglichst knapp bemessen und beträgt bevorzugt weniger als 50cm, noch weiter bevorzugt weniger als 40cm, nochweiter bevorzugt weniger als 30cm, noch weiter bevorzugt weniger als 20cm oder als 10 cm, ganz besonders bevorzugt weniger als 5cm, gemessen in Strömungsrichtung der Dekontaminationsflüssigkeit aus der Ringleitung hin zum jeweiligen Zerstäuber. Bevorzugt wird die Leitungslänge bis gegen null reduziert. Denkbar und bevorzugt ist es für den Fall des Vorsehens einer Leitungslänge zwischen jeweiligem Ventil und zugehörigen Zerstäuber diese Leitung, insbesondere am Ende eines Dekontaminationsvorgangs (bevorzugt nach einem Wiegevorgang der Dekontaminationsflüssigkeit im Vorratsbehälter) mit Druckluft aus der Ringleitung in den zu dekontaminierenden Raum über den jeweiligen Zerstäuber leerzudrücken.

Bevorzugt wird mit der Dekontaminationsanordnung und/oder dem Dekontaminationsverfahren während eines Dekontaminationsvorgang zwischen 5g und 10g, insbesondere 7,5g, Dekontaminationsflüssigkeit pro Kubikmeter zu dekontaminierendem Raum über die Gesamtheit der Zerstäuber aus der Ringleitung in den zu dekontaminierenden Raum zerstäubt.

Bevorzugt ist die Dekontaminationsanordnung derart ausgebildet bzw. wird das Dekontaminationsverfahren derart durchgeführt, dass während eines Dekontaminationszyklus, d.h. während die Dekontaminationsflüssigkeit an die Zerstäuber über die Ringleitung abgegeben wird, zeitabschnittsweise oder durchgehend Dekontaminationsflüssigkeit über die Ringleitung zurück in den Dekontaminationsflüssigkeitsvorratsbehälter strömt, d.h. im Kreislauf befördert wird.

Grundsätzlich ist es möglich und im Rahmen der Erfindung vorgesehen, dass die Dekontaminationsanordnung mehrere zu dekontaminierende Räume aufweist. Dabei ist es möglich, einzelnen Räumen jeweils eine Ringleitung mit diesen zugeordneten Zerstäubern zuzuordnen. Auch ist eine alternative Ausführungsform realisierbar, bei der die eine Ringleitung durch mehrere zu dekontaminierende Räume geführt ist, derart, dass in den unterschiedlichen zu dekontaminierenden Räumen angeordnete Zerstäuber über diese gemeinsame Ringleitung mit Dekontaminationsflüssigkeit aus dem Dekontaminationsflüssigkeitsbehälter versorgbar sind bzw. versorgt werden. Auch ist eine Ausführungsform realisierbar, bei der nur einem einzigen zu dekontaminierenden Raum eine Ringleitung zugeordnet ist, mit der mehrere in dem zu dekontaminierenden Raum angeordnete Zerstäuber mit Dekontaminationsflüssigkeit versorgbar sind bzw. versorgt werden.

Wie bereits angedeutet lässt sich mit der erfindungsgemäßen Dekontaminationsanordnung und dem erfindungsgemäßen Dekontaminationsverfahren die Gesamtmenge von an die Zerstäuber während eines Dekontaminationszyklus abgegebener Dekontaminationsflüssigkeit einfach und schnell bestimmen. Erfindungsgemäß umfasst die Dekontaminationsanordnung bzw. kommen hierzu im Rahmen des Verfahrens entsprechende Messmittel zum Einsatz. Bevorzugt umfassen die Messmittel dabei Wägemittel, insbesondere in Form einer Waage, zum Bestimmen einer Dekontaminationsflüssigkeitsmenge im Dekontaminationsflüssigkeitsvorratsbehälter. Im einfachsten Fall kann die Gesamtmenge an abgegebener Dekontaminationsflüssigkeit manuell bestimmt werden, indem ein Messergebnis (Gewicht) des Dekontaminationsflüssigkeitsvorratsbehälters (jeweils bei befüllter Ringleitung) vor und nach einem Dekontaminationsflüssigkeitszyklus abgelesen und die Differenz gebildet wird oder indem die Wägemittel bei gefüllter Ringleitung vor einem Dekontaminationsflüssigkeitszyklus auf Null gesetzt werden (Tara) und unmittelbar die abgegebene Dekontaminationsflüssigkeitsmenge bzw. deren Gewicht nach einem Dekontaminationszyklus bei gefüllter Ringleitung abgelesen wird. Alternativ ist es möglich und bevorzugt die Messmittel entsprechend automatisiert auszubilden, wobei die Messmittel dann die Gesamtmenge von an die Zerstäuber abgegebener Dekontaminationsflüssigkeit bestimmend ausgebildet sind durch Differenzbildung aus dem Gewicht der Dekontaminationsflüssigkeitsmenge im Dekontaminationsflüssigkeitsvorratsbehälter bei mit Dekontaminationsflüssigkeit befüllter Ringleitung vor dem Dekontaminationszyklus und dem Gewicht der Dekontaminationsflüssigkeitsmenge in Dekontaminationsflüssigkeitsvorratsbehälter bei mit Dekontaminationsflüssigkeit gefüllter Ringleitung nach dem Dekontaminationszyklus.

Im Hinblick auf die Ausgestaltung der Druckregelmittel gibt es unterschiedliche Möglichkeiten. Grundsätzlich ist der Einsatz einer elektronischen Regelstrecke möglich, bei der ein Ist-Druck in der Ringleitung über entsprechende Messmittel gemessen wird und dieser Ist-Zustand in eine entsprechende Regelung hin zu einem Soll-Druck einfließt, wobei die Regelstrecke bevorzugt als Stellglied ein entsprechend ansteuerbares bzw. angesteuertes Proportionalventil, insbesondere ein elektromagnetisches Proportionalventil oder ein getaktet betriebenes, insbesondere PWM-angesteuertes Schaltventil umfasst, über welches zur Druckbegrenzung Dekontaminationsflüssigkeit zum Dekontaminationsflüssigkeitsbehälter abgesteuert wird. Zusätzlich oder alternativ ist auch bei entsprechender Ausbildung und Ansteuerung der Pumpe eine Druckregelung über eine entsprechende Pumpenregelung möglich. Insbesondere bei der Realisierung einer Pumpenregelung ist es möglich, auf eine Absteuerung von Dekontaminationsflüssigkeit während des Dekontaminationszyklus in Richtung Dekontaminationsflüssigkeitsvorratsbehälter zu verzichten, wobei selbstverständlich auch eine Kombination aus Pumpenregelung und Absteuerung realisierbar ist. Wie im Folgenden noch erläutert werden wird ist eine Absteuerung (mit oder bevorzugt ohne Pumpenregelung) von Dekontaminationsflüssigkeit in den Dekontaminationsflüssigkeitsvorratsbehälter während des Dekontaminationszyklus auch mit einem mechanisch arbeitenden Rückschlagventil bzw. Absteuerventil möglich.

Ganz besonders bevorzugt ist jedoch eine Ausführungsform, bei der die Druckregelmittel ein, insbesondere in der Ringleitung, ganz besonders bevorzugt endseitig bzw. den Abzweigungen für die Zerstäuber in Strömungsrichtung der Dekontaminationsflüssigkeit in der Ringleitung nachgeordnetes, Rückschlagventil umfassen, dessen Ventilelement das Rückschlagventil bzw. die Ringleitung in Richtung Dekontaminationsflüssigkeitsvorratsbehälter entgegen der Federkraft einer Rückstellfeder öffnend ausgebildet ist, um auf diese Weise zur Druckeinstellung bzw. -begrenzung Dekontaminationsflüssigkeit in den Dekontaminationsflüssigkeitsvorratsbehälter abzusteuern. Anders ausgedrückt umfassen die Druckregelmittel ein mechanisch arbeitendes und damit robustes sowie ein sehr schnell reagierendes Rückschlagventil, welches im Zusammenspiel mit der einen Förderdruck erzeugenden (Förder-)Pumpe den Flüssigkeitsdruck in der Ringleitung automatisch regelt, insbesondere indem es den Flüssigkeitsdruck auf einen Maximalwert begrenzt, indem der Öffnungsgrad des Rückschlagventils in Richtung Dekontaminationsflüssigkeitsvorratsbehälter variiert wird. Eine zusätzliche Regelung des Fördervolumens der Pumpe ist dabei nicht notwendig (und bevorzugt auch nicht realisiert) - bevorzugt wird diese mit einer gleichbleibenden elektrischen Leistung versorgt bzw. betrieben.

Insgesamt ist es bevorzugt, insbesondere bei einer Ausführung der Druckregelmittel als Rückschlagventil, wenn die Druckregelmittel derart ausgebildet und auf die Pumpe, insbesondere auf einer konstant bleibenden Pumpenleistung abgestimmt sind, bevorzugt durch Einstellung und/oder Vorgabe (Dimensionierung) des Arbeitspunktes der Druckregelmittel, insbesondere des mechanisch arbeitenden Rückschlagventils, dass bei geöffneten Ventilmitteln, also während der Abgabe von Dekontaminationsflüssigkeit über die Zerstäuber an den zu dekontaminierenden Raum Dekontaminationsflüssigkeit durch den Auslass der Ringleitung zurück in den Dekontaminationsflüssigkeitsvorratsbehälter strömt, d.h. abgesteuert wird. Hierdurch können Druckschwankungen, wie sie bei einem vollständigen Schließen der Druckregelmittel auftreten würden, sicher verhindert werden.

Wie eingangs bereits angedeutet ermöglicht das erfindungsgemäße Vorsehen der Druckeinstellmittel in Kombination mit der Ringleitung die Abgabemenge an Dekontaminationsflüssigkeit über die Zerstäuber mithilfe einfach aufgebauter und robuster Schaltventile, insbesondere 2/2-Wegeventile einzustellen bzw. vorzugeben, indem die Öffnungs- und Geschlossenzeit der Schaltventile bzw. Ventilmittel über die Steuermittel vorgegeben wird. Ganz besonders bevorzugt ist es, wenn einzelnen Zerstäubern, bevorzugt sämtlichen Zerstäubern jeweils ein solches Schaltventil zur Versorgung des jeweiligen Zerstäubers mit Dekontaminationsflüssigkeit aus der Ringleitung zugeordnet sind. Für den Fall des Zusammenschlusses mehrerer Zerstäuber zu einer Gruppe ist es alternativ auch denkbar dieser Gruppe von Zerstäubern ein, insbesondere einziges Schaltventil zuzuordnen. Für den Fall des Vorsehens mehrerer Gruppen ist bevorzugt jeder Gruppe, insbesondere sämtlichen Gruppen ein solches Schaltventil zugeordnet und zwischen den Zerstäubern und der Ringleitung angeordnet. Eine optimale Einflussnamemöglichkeit auf die Aerosolbildung und die abgegebene Dekontaminationsflüssigkeitsmenge pro Zerstäuber ist dann gegeben, wenn jedem Zerstäuber ein solches Schaltventil zugeordnet ist.

Grundsätzlich ist es möglich, mehrere oder einzelne Schaltventile, insbesondere sämtliche Schaltventile während eines gesamten Dekontaminationszyklus geöffnet zu lassen oder nur über einen Teilabschnitt (Zeitabschnitt) oder mehrere zeitlich beabstandete Zeitabschnitte des Dekontaminationszyklus. Ganz besonders bevorzugt ist es jedoch, wenn mindestens eines der Schaltventile, insbesondere mehrere Schaltventile, noch weiter bevorzugt sämtliche Schaltventile intermittierend (getaktet bzw. pulsierend) betrieben bzw. von den Steuermitteln angetrieben werden. Grundsätzlich ist es möglich, sämtliche Schaltventile gleich anzusteuern - bevorzugt ist eine Ausführungsform, bei der mindestens zwei der Schaltventile unterschiedlich angesteuert werden, d.h. mit voneinander unterschiedlichen Öffnungs- und Schließzeiten, um eine unterschiedliche Abgabemenge an Dekontaminationsflüssigkeit über die den Schaltventilen zugeordneten Zerstäubern zu bewirken, insbesondere um besonderes kritische bzw. infektionsanfällige Bereiche des zu dekontaminierenden Raums mit einer größeren Menge an Dekontaminationsflüssigkeitsaerosol zu beaufschlagen. Auch ist es möglich mehrere Schaltventile oder Gruppen von Schaltventilen nacheinander, insbesondere gepulst zu betreiben bzw. zu öffnen, um somit eine Aerosolströmung im zu dekontaminierenden Raum zu bewirken, wodurch eine noch bessere Verteilung des Dekontaminationsflüssigkeitsaerosols in dem zu dekontaminierenden Raum gewährleistet werden kann.

Wie eingangs bereits erwähnt ist es bevorzugt, wenn der Abstand zwischen den Zerstäubern und der Ringleitung, also die jeweilige Verbindungsleitungslänge möglichst kurz bemessen ist und/oder bei sämtlichen Zerstäubern, zumindest näherungsweise gleich. Ganz besonders bevorzugt sind die Schaltventile unmittelbar benachbart zur Ringleitung angeordnet und die Zerstäuber schließen wiederum unmittelbar an das jeweils zugehörige Schaltventil an.

Im Hinblick auf die konkrete Ausgestaltung der Zerstäuber gibt es unterschiedliche Möglichkeiten. So ist es grundsätzlich denkbar Ultraschallzerstäuber oder auf anderen physikalischen Wirkprinzipien arbeitende Zerstäuber einzusetzen.

Erfindungsgemäß sind jedoch als Zerstäuber Zweistoffdüsen (Mischdüsen) einzusetzen, die mit einer Gasdruckquelle, insbesondere einer Druckluftquelle zum Erzeugen eines Trägergasstroms verbunden sind, wobei dem Trägergasstrom über die Ventilmittel, insbesondere das zugehörige Schaltventil, die zum Erzeugen des gewünschten Aerosols notwendige Dekontaminationsflüssigkeitsmenge, insbesondere gepulst (getaktet) bzw. intermittierend zudosiert wird.

Alternativ ist es inbesondere für kleinere zu dekontaminierende Räume möglich Einstoffdüsen einzusetzen, die bevorzugt auch gepulst betrieben bzw. mit Dekontaminationsflüssigkeit versorgt werden. Bevorzugt sind die Zerstäuber jedenfalls derart ausgebildet, dass 90% der die Zerstäuber verlassenden Dekontaminationsnebel bzw. -aerosoltröpfen eine Partikelgröße von kleiner als 20µm, bevorzugt kleiner als 10µm aufweisen.

Besonders bevorzugt ist es, wenn der Pumpe nachgeordnet ein Verbindungsventil in der Ringleitung vorgesehen ist, um bei geöffnetem Verbindungsventil zwei Ringleitungsabschnitte byzupassen. Auf diese Weise ist es möglich, Förderdruck von zwei Seiten der Ringleitung über die Pumpe in die Ringleitung und somit in Richtung der Zerstäuber aufzubringen, was für noch gleichmäßigere Druckverhältnisse Sorge trägt. Bevorzugt mündet die Bypassleitung den Druckeinstellmittel vorgeordnet wieder in einen endseitigen Bereich der Ringleitung in diese ein. Anders ausgedrückt schließt die Bypassleitung einen Ringleitungsabschnitt, an dem die Zerstäuber über die Ventilmittel unmittelbar angeschlossen sind, kurz.

Unabhängig davon ist es bevorzugt, wenn die Druckeinstellmittel über ein Bypassventil bypassbar sind, um die Ringleitung verbessert spülen und/oder mit Druckluft leerdrücken zu können. Unabhängig davon ist der Ringleitung bevorzugt ein Druckgasanschluss, insbesondere ein Druckluftanschluss zum Leerdrücken der Ringleitung zugeordnet. Der Druckluftanschluss kann auch genutzt werden, um, insbesondere vor einem Dekontaminationszyklus die Ringleitung auf Leckage zu testen, indem die Ringleitung Druckgas, insbesondere Druckluft beaufschlagt wird und ein etwaiger Druckabfall überwacht wird, insbesondere durch eine entsprechende, der Ringleitung zugeordnete Drucküberwachungseinrichtung.

Die Erfindung führt auch auf ein Isolatorsystem mit einer zuvor beschriebenen Dekontaminationsanordnung, wobei der zu dekontaminierende Raum ein Raum dieses Isolators ist, insbesondere ein Manipulatorraum und/oder ein von dem Manipulatorraum über eine Membran zur Laminarisierung eines Umluftstroms eines Umluftgebläses getrennten Plenumraum. Bevorzugt befindet sich im Manipulatorraum eine Handhabungseinrichtung zum Handhaben, insbesondere Herstellen, Dosieren und/oder Abfüllen eines pharmazeutischen bzw. medizinischen Wirkstoffs.

Des Weiteren führt die Erfindung auf ein Dekontaminationsverfahren. Erfindungsgemäß ist dabei vorgesehen, die fluidleitend über die Ventilmittel, insbesondere Schaltventile, mit einer einen Einlass und einen davon separaten Auslass aufweisenden Ringleitung verbunden sind, mit Dekontaminationsflüssigkeit versorgt werden, indem in den Einlass Dekontaminationsflüssigkeit aus dem Dekontaminationsflüssigkeitsvorratsbehälter mittels einer Pumpe gefördert wird und über den Auslass, insbesondere während eines Dekontaminationszyklus, d.h. während einer Abgabe von Dekontaminationsflüssigkeit über die Zerstäuber, Dekontaminationsflüssigkeit zurück in den Dekontaminationsflüssigkeitsvorratsbehälter strömt, wobei ein Flüssigkeitsdruck der Dekontaminationsflüssigkeit in der Ringleitung über Druckeinstellmittel in einem vorgegebenen Bereich gehalten, insbesondere auf einen maximalen Druck begrenzt wird, wobei die Pumpe für die entsprechende Druckbeaufschlagung Sorge trägt.

Vor Beginn eines Dekontaminationszyklus wird die Ringleitung, falls sie zuvor, beispielsweise mit Druckluft, entleert wurde, was bevorzugt ist, vollständig mit Dekontaminationsflüssigkeit aus dem Dekontaminationsflüssigkeitsvorratsbehälter gefüllt.

Bevorzugt werden im Rahmen des Verfahrens die weiter bevorzugt als Schaltventile ausgebildeten Ventilmittel über die Steuermittel zeitgesteuert angesteuert, derart, dass die Offen- und/oder Geschlossenzeit der Schaltventile während eines Dekontaminationszyklus vorgegeben wird, um somit wiederum die Menge an über die Zerstäuber abzugebender Dekontaminationsflüssigkeit vorzugeben. Bevorzugt werden die Schaltventile dabei getaktet, d.h. intermittierend betrieben, also derart, dass diese alternierend zwischen einem geschlossenen und einem, bevorzugt vollständig, geöffneten Schaltzustand umschalten. Durch Zuweisung einer bestimmten Öffnungs- und/oder Schließzeit zu den einzelnen Ventilen der Ventilmittel kann eine gewünschte, insbesondere gleichmäßige Verteilung des Desinfektionsflüssigkeitsaerosols sichergestellt werden.

Insbesondere ist es auch möglich einzelne Zerstäuber oder Gruppen von Zerstäubern periodisch zu betreiben, insbesondere in jeder aktiven Periode wiederum getaktet bzw. intermittierend, um so die Verteilung zu beeinflussen. Die Verteilung des Aerosols zu beeinflussen.

Bevorzugt ist während des Dekontaminationszyklus eine Gas- bzw. Luftdruckregulierungseinrichtung betreffend den Gasdruck, insbesondere Luftdruck im zu dekontaminierenden Raum aktiv, um somit für konstante Gasdruckverhältnisse während der Dekontamination Sorge zu tragen.

Weitere bevorzugte Ausführungsformen und Details des Verfahrens sind im Zusammenhang mit der Beschreibung der Dekontaminationsanordnung sowie des Isolatorsystems beschrieben.

Weitere Vorteile, Merkmale und Einzelheiten der Erfindung ergeben sich aus der nachfolgenden Beschreibung eines bevorzugten Ausführungsbeispiels sowie anhand der Zeichnung.

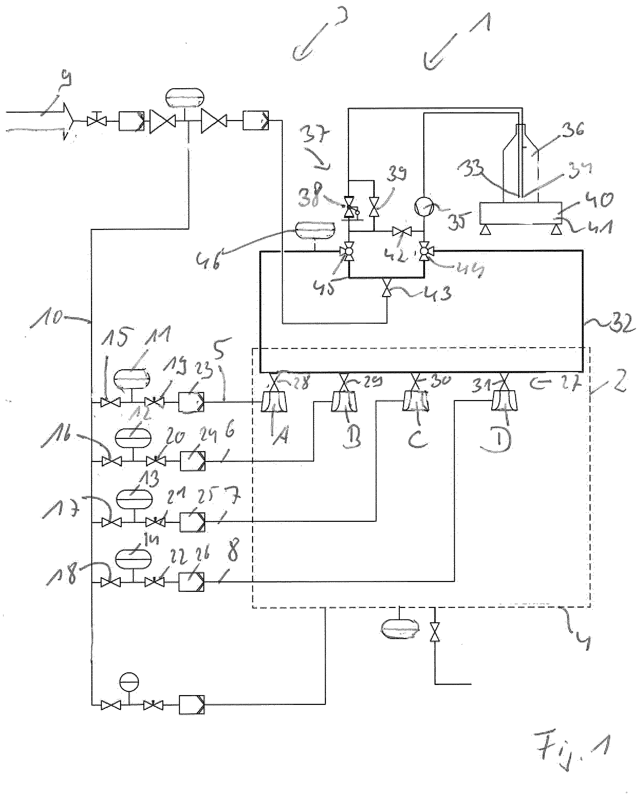

Diese zeigt in der einzigen Fig. 1 ein Fluidschema eines bevorzugten Ausführungsbeispiels einer nach dem Konzept der Erfindung ausgebildeten Dekontaminationsanordnung, bei der der zu dekontaminierende Raum ein Isolatorraum, vorliegend bevorzugt ein Manipulatorraum ist.

In Fig. 1 ist ein Isolatorsystem 1 mit einem Isolator 2 für pharmatechnische Anwendungen sowie einer dem Isolator zugeordneten bzw. in diesen integrierten Dekontaminationsanordnung 3 zur Dekontamination eines zu dekontaminierenden Raums 4 gezeigt, bei welchem es sich vorliegend um einen sog. Manipulatorraum des Isolators 2 handelt, indem ein pharmatechnischer Prozess, beispielsweise ein Dosier-, Herstellungs- und/oder Abfüllprozess mit entsprechender Maschinerie bzw. Apparatur abläuft, wobei in den Manipulatorraum über nicht gezeigte Handschuhports manuell eingegriffen werden kann. Der Manipulatorraum ist in an sich bekannter Weise über Rückströmkanäle mit einem oberhalb des zu dekontaminierenden Raums angeordneten Umlufterzeugerraum angeordnet, in welchem sich ein Umluftgebläse befindet, über welches im Betrieb des Isolators 2 ein Umluftvolumenstrom erzeugbar ist und zwar von dem Umluftraum über entsprechende Hochleistungsschwebstofffilter in einen sog. Plenumraum hinein und über diesen durch eine Membran hindurch in den Manipulatorraum. Die vorerwähnten Rückströmkanäle sind dabei bevorzugt zwischen zwei transparenten Scheiben, insbesondere durchsichtigen Glasscheiben angeordnet.

Die Dekontaminationsanordnung umfasst vorliegend mehrere Zerstäuber A, B, C, D, die jeweils als Zweistoffdüse (Mischdüse) ausgebildet sind. Zu diesem Zweck ist jeder Zerstäuber A, B, C, D an eine zugehörige Druckluftversorgungsleitung 5, 6, 7, 8 angeschlossen, die wiederum mit Druckluft aus einer Druckluftquelle 9 versorgbar sind. Die Druckluftversorgungsleitungen 5, 6, 7, 8 sind vorliegend an einer Druckluftverteilerleitung 10 angeschlossen, über die auch der zu dekontaminierende Raum 4 selbst mit hochreiner, konditionierter (temperierter und/oder feuchtigkeitsregulierter) Druckluft versorgbar ist.

Vorliegend ist jeder Druckluftversorgungsleitung 5, 6, 7, 8 eine Druckmesseinrichtung 11, 12, 13, 14 zugeordnet, um den jeweils anstehenden Luftdruck überwachen zu können. Ferner ist in jeder Druckluftversorgungsleitung 5, 6, 7, 8 ein Sperrventil 15, 16, 17, 18 angeordnet, um eine Verbindung zwischen einem jeweiligen Zerstäuber A, B, C, D und der Druckluftquelle 9 absperren oder öffnen zu können. Der an den einzelnen Zerstäubern A, B, C, D anliegende Luftdruck (Versorgungsluftdruck) ist über entsprechende Drosseln bzw. Einstellventile 19, 20, 21, 22 vorgebbar bzw. vorgegeben. Ferner befindet sich in jeder Druckluftversorgungsleitung 5, 6, 7, 8 ein Filter 23, 24, 25, 26. Jedenfalls wird den Zerstäubern A, B, C, D ein Druckluft-Trägerluftstrom zur Verfügung gestellt, in den dann, wie später noch erläutert werden wird, Dekontaminationsflüssigkeit, bevorzugt intermittierend zudosiert wird, wobei diese Dekontaminationsflüssigkeit mittels der Zerstäuber A, B, C, D, die sich in dem zu dekontaminierenden Raum 4 befinden feinstverteilt bzw. vernebelt wird, indem ein entsprechendes Dekontaminationsflüssigkeitsaerosol erzeugt wird.

Den Zerstäubern A, B, C, D sind Ventilmittel 27 vorgeordnet und zwar vorliegend in der Form von Schaltventilen 28, 29, 30, 31, die über nicht gezeigte Steuermittel ansteuerbar sind und zwar zeitgesteuert zur Beeinflussung der jeweiligen Öffnungs- und Schließzeit, um somit wiederum die Menge an die Zerstäuber A, B, C, D abgegebene Dekontaminationsflüssigkeitsmenge vorzugeben. Dabei werden die Schaltventile 28, 29, 30, 31 im vorliegenden Ausführungsbeispiel intermittierend, d.h. getaktet betrieben. Die Gesamtöffnungszeit der einzelnen Schaltventile 28, 29, 30, 31 wird über Steuermittel zur Vorgabe der abzugebenden Dekontaminationsflüssigkeitsmenge vorgegeben. In der Darstellung gemäß Fig. 1 ist zu erkennen, dass die Zerstäuber A, B, C, D unmittelbar an die Schaltventile 28, 29, 30, 31 angrenzen. Die Schaltventile 28, 29, 30, 31 wiederum grenzen unmittelbar an eine Ringleitung 32 an bzw. steuern die Dekontaminationsflüssigkeitsabgabe aus dieser Ringleitung an den jeweils zugeordneten Zerstäuber.

Wie im allgemeinen Beschreibungsteil erläutert ist, können die Schaltventile 28, 29, 30, 31 alle gleich angesteuert werden oder unterschiedlich, beispielsweise zeitlich beabstandet nacheinander oder überschneidend, um somit auch die Dekontaminationsaerosolströmung zu dekontaminierenden Raum 4 beeinflussen zu können.

Die Ringleitung 32 umfasst einen Einlass 33 sowie einen davon separaten Auslass 34, wobei in den Einlass 33 Dekontaminationsflüssigkeit über eine Pumpe 35 gefördert wird und zwar aus einem Dekontaminationsflüssigkeitsvorratsbehälter 36. Auf diese Weise gelangt Dekontaminationsflüssigkeit zu den Schaltventilen 28, 29, 30, 31 und über diese zu dem jeweils zugeordneten Zerstäuber A, B, C, D. Über den Auslass 34 strömt Dekontaminationsflüssigkeit zurück den Dekontaminationsflüssigkeitsvorratsbehälter 36, wird also im Kreislauf gefördert.

Der Ringleitung 32 sind Druckeinstellmittel 37 zugeordnet, vorliegend umfassend ein mechanisch arbeitendes Rückschlagventil 38, welches so auf die, insbesondere konstante Förderleistung der Pumpe 35 abgestimmt ist, dass während eines Dekontaminationszyklus, bei dem Dekontaminationsflüssigkeit über zumindest einen der Zerstäuber A, B, C, D in ein Aerosol umgewandelt wird in den Dekontaminationsflüssigkeitsvorratsbehälter 36 zurückströmt.

Während des Dekontaminationszyklus ist ein Bypassventil für ein mögliches Bypassen des Rückschlagventils 38 geschlossen.

Dem Dekontaminationsflüssigkeitsvorratsbehälter 36 sind Messmittel 40, vorliegend in Form von Wägemitteln 41 zugeordnet, mit denen das Gewicht der im Dekontaminationsflüssigkeitsvorratsbehälter 36 befindlichen Dekontaminationsflüssigkeit bei gefüllter Ringleitung 32 mit Dekontaminationsflüssigkeit vor einem Dekontaminationszyklus bestimmbar ist sowie das Gewicht der im Dekontaminationsflüssigkeitsvorratsbehälter 36 befindlichen Dekontaminationsflüssigkeit nach Beendigung des Dekontaminationszyklus, ebenfalls bei gefüllter Ringleitung 32.

Fakultativ ist ein Verbindungsventil 42 vorgesehen, mit dem ein Anfangsbereich und ein Endbereich der Ringleitung 32 verbindbar sind, sodass insbesondere während eines Dekontaminationszyklus Dekontaminationsflüssigkeit mittels der Pumpe 35 von zwei Seiten her in einen Abschnitt der Ringleitung 32 förderbar ist, von welchem aus die Zerstäuber A, B, C, D versorgbar sind bzw. an welchen die Zerstäuber A, B, C, D über die Ventilmittel 27 angeschlossen sind.

Der Ringleitung 32 ist ferner ein Druckluftanschluss 43 zugeordnet, um die Ringleitung 32 leer drücken zu können, wozu die Leitungsverbindungen mit vorgesehenen 3/2-Wegeventile 44, 45 entsprechend schaltbar sind.

Zu erkennen ist, dass der Ringleitung 32 eine Fluiddruckmesseinrichtung 46 zugeordnet ist. Diese dient vorliegend lediglich der Überwachung des Flüssigkeitsdrucks in der Ringleitung 32 und ist nicht Bestandteil einer Druckregelung - vorliegend erfolgt die Druckeinstellung des Fluiddrucks ausschließlich durch ein Zusammenspiel von Pumpe 35 und Rückschlagventil 38, wobei alternative Lösungen zur Druckeinstellung bzw. zum Halten des Flüssigkeitsdrucks in einem gewünschten Bereich realisierbar sind, wie im allgemeinen Beschreibungsteil erläutert ist.

## Patentansprüche

1. Dekontaminationsanordnung, insbesondere für pharmatechnische Anwendungen, umfassend mindestens einen zu dekontaminierenden Raum (4), insbesondere einen Isolatorraum, bevorzugt einen Manipulatorraum und/oder einen von dem Manipulatorraum über eine Membran getrennten Plenumraum, mit einem Dekontaminationsflüssigkeitsvorratsbehälter (36) aus dem mehrere in dem zu dekontaminierenden Raum (4) angeordnete, bevorzugt als Zweistoffdüsen ausgebildete, Zerstäuber (A, B, C, D) zum Erzeugen eines Dekontaminationsmittelaerosols über mittels elektronische Steuermittel ansteuerbare Ventilmittel (27) mit Dekontaminationsflüssigkeit, bevorzugt Wasserstoffperoxid, versorgbar sind, wobei die Zerstäuber (A, B, C, D) fluidleitend über die Ventilmittel (27) mit einer einen Einlass (33) und einem davon separaten Auslass (34) aufweisenden Ringleitung (32) verbunden sind, in deren Einlass Dekontaminationsflüssigkeit aus dem Dekontaminationsflüssigkeits-vorratsbehälter (36) mittels einer Pumpe (35) förderbar ist und aus deren Auslass Dekontaminationsflüssigkeit zurück in den Dekontaminationsflüssigkeitsvorratsbehälter (36) strömen kann, und dass der Ringleitung (32) Druckeinstellmittel (37) zum Halten des Flüssigkeitsdrucks in der Ringleitung (32) in einem vorgegebenen Flüssigkeitsdruckbereich zugeordnet sind,
**dadurch gekennzeichnet,**
**dass** Messmittel (40) zur Bestimmung einer Gesamtmenge von an die Zerstäuber (A, B, C, D) während eines Dekontaminationszyklus abgegebener Dekontaminationsflüssigkeit vorgesehen sind, insbesondere umfassend Wägemittel (41) zum Bestimmen einer Dekontaminationsflüssigkeitsmenge im Dekontaminationsflüssigkeits-vorratsbehälter (36) und dass die als Zweistoffdüsen ausgebildeten Zerstäuber (A, B, C, D) gasleitend mit einer Gasdruckquelle, insbesondere einer Druckluftquelle (9) zum Erzeugen eines Trägergasstroms für das zu erzeugende Dekontaminationsmittelaerosols verbunden sind.

2. Dekontaminationsanordnung nach Anspruch 1,
**dadurch gekennzeichnet,**
**dass** die Messmittel (40) die Gesamtmenge von an die Zerstäuber abgegebener Dekontaminationsflüssigkeit bestimmend ausgebildet sind durch Differenzbildung aus dem Gewicht der Dekontaminations- flüssigkeitsmenge im Dekontaminationsflüssigkeits-vorratsbehälter (36) bei mit Dekontaminationsflüssigkeit gefüllter Ringleitung (32) vor dem Dekontaminationszyklus und dem Gewicht der Dekontaminationsflüssigkeitsmenge im Dekontaminations-flüssigkeitsvorratsbehälter (36) bei mit Dekontaminationsflüssigkeit gefüllter Ringleitung (32) nach dem Dekontaminationszyklus.

3. Dekontaminationsanordnung nach einem der vorhergehenden Ansprüche,
**dadurch gekennzeichnet,**
**dass** die Druckeinstellmittel (37) ein, insbesondere in der der Ringleitung (32) angeordnetes, Rückschlagventil (38) umfassen, dessen Ventilelement das Rückschlagventil (38) in Richtung Dekontaminationsflüssigkeitsvorratsbehälter (36) entgegen der Federkraft einer Rückstellfeder öffnend ausgebildet ist.

4. Dekontaminationsanordnung nach einem der vorhergehenden Ansprüche
**dadurch gekennzeichnet,**
**dass** die Druckeinstellmittel (37) derart ausgebildet und auf die Pumpe (35) abgestimmt sind, dass bei geöffneten Ventilmitteln (27) Dekontaminationsflüssigkeit durch den Auslass (34 zurück in den Dekontaminationsflüssigkeitsvorratsbehälter (36) strömt.

5. Dekontaminationsanordnung nach einem der vorhergehenden Ansprüche,
**dadurch gekennzeichnet,**
**dass** die Ventilmittel (27), insbesondere getaktet ansteuerbare, Schaltventile (28, 29, 30, 31), insbesondere 2-/2-Wegeventile umfassen, wobei bevorzugt einzelnen Zerstäubern (A, B, C, D), insbesondere sämtlichen Zerstäubern (A, B, C, D) jeweils ein solches Schaltventil (28, 29, 30, 31) zur Versorgung des jeweiligen Zerstäubers (A, B, C, D) mit Dekontaminationsflüssigkeit aus der Ringleitung (32), zugeordnet sind und/oder dass mindestens einer Gruppe von Zerstäubern (A, B, C, D), bevorzugt mehreren Gruppen von Zerstäubern (A, B, C, D) jeweils ein solches Schaltventil (28, 29, 30, 31) zugeordnet ist/sind.

6. Dekontaminationsanordnung nach Anspruch 5,
**dadurch gekennzeichnet,**
**dass** die Steuermittel die Schaltventile (28, 29, 30, 31) derart ansteuernd ausgebildet sind, dass mehre, insbesondere sämtliche Schaltventile (28, 29, 30, 31) oder Schaltventilgruppen gleichzeitig geöffnet sind und/oder getaktet betrieben werden und/oder derart dass mehrere Schaltventile (28, 29, 30, 31) oder Schaltventilgruppen zeitlich nacheinander geöffnet sind und/oder getaktet betrieben sind und/oder derart, dass mindestens eines der Schaltventile (28, 29, 30, 31) oder mindestens eine der Schaltventilgruppen bei einem Dekontaminationszyklus länger geöffnet ist und/oder länger getaktet betrieben wird als mindestens ein anderes der Schaltventile (28, 29, 30, 31) oder eine andere der Schaltventilgruppen.

7. Dekontaminationsanordnung nach einem der Ansprüche 5 oder 6,
**dadurch gekennzeichnet,**
**dass** die Steuermittel die Schaltventile (28, 29, 30, 31) derart ansteuernd ausgebildet sind, dass eine vorgegebene zu zerstäubende Gesamtmenge an Dekontaminationsflüssigkeit durch Steuerung einer, insbesondere getakteten, Gesamtöffnungszeit der Schaltventile (28, 29, 30, 31) dosierbar ist und/oder derart, dass eine vorgegebene an einem der Zerstäuber (A, B, C, D) oder einer Gruppe der der Zerstäuber (A, B, C, D) zu dosierende Menge an Dekontaminationsflüssigkeit durch Steuerung einer, insbesondere getakteten, Öffnungszeit des zugehörigen Schaltventils (28, 29, 30, 31) dosierbar ist.

8. Dekontaminationsanordnung nach einem der vorhergehenden Ansprüche,
**dadurch gekennzeichnet,**
**dass** mehrere an die Ringleitung (32) angeschlossene Zerstäuber (A, B, C, D), bevorzugt die meisten an die Ringleitung (32) angeschlossene Zerstäuber (A, B, C, D), bevorzugt sämtliche an die Ringleitung (32) angeschlossene Zerstäuber (A, B, C, D) äquidistant von der Ringleitung (32) beabstandet sind.

9. Dekontaminationsanordnung nach einem der vorhergehenden Ansprüche,
**dadurch gekennzeichnet,**
**dass** der Ringleitung (32) nach der Pumpe (35) ein Verbindungsventil (42) zum Herstellen und Schließen eines Bypasses zwischen einem Bereich der Ringleitung (32) vor und nach den Zerstäubern (A, B, C, D) zugeordnet ist und/oder dass die Druckeinstellmittel (37) über ein Bypassventil (39) bypassbar sind und/oder dass der Ringleitung (32) ein Druckgasanschluss, insbesondere ein Druckluftanschluss (43) zum Entleeren der Ringleitung (32) zugeordnet ist.

10. System, umfassend einen Isolator (2), bevorzugt eingerichtet für pharmatechnische Anwendungen, mit einem zu dekontaminierenden Raum (4), insbesondere einem Manipulatorraum und/oder einem von dem Manipulatorraum über eine Membran zur Laminarisierung eines Luftstroms eines Umluftgebläses getrennten Plenumraum, sowie mit einer Dekontaminationsanordnung (3) nach einem der vorhergehenden Ansprüche.

11. Dekontaminationsverfahren wobei ein zu dekontaminierender Raum (4), insbesondere ein Isolatorraum, bevorzugt ein Manipulatorraum und/oder ein von dem Manipulatorraum über eine Membran getrennter Plenumraum, dekontaminiert wird, indem mittels in dem zu dekontaminierenden Raum (4) angeordnete, bevorzugt als mit Druckluft versorgte Zweistoffdüsen ausgebildete, Zerstäuber (A, B, C, D) ein Dekontaminationsmittelaerosol aus einer Dekontaminations-flüssigkeit, insbesondere Wasserstoffperoxid, erzeugt wird, die mit der Dekontaminationsflüssigkeit aus einem Dekontaminationsflüssigkeitsvorratsbehälter (36) über Ventilmittel (27) versorgt werden, wobei die Zerstäuber (A, B, C, D) fluidleitend über die Ventilmittel (27) mit einer einen Einlass (33) und einem davon separaten Auslass (34) aufweisenden Ringleitung (32) verbunden sind in deren Einlass Dekontaminationsflüssigkeit aus dem Dekontaminations-flüssigkeitsvorratsbehälter (36) mittels einer Pumpe (35) gefördert wird und aus deren Auslass, insbesondere während einer Abgabe von Dekontaminationsflüssigkeit über die Zerstäuber (A, B, C, D), Dekontaminationsflüssigkeit zurück in den Dekontaminations-flüssigkeitsvorratsbehälter (36) strömt und dass ein Flüssigkeitsdruck der Dekontaminationsflüssigkeit in der Ringleitung (32) über Druckeinstellmittel (37) in einem vorgegebenen Druckbereich gehalten wird,
**dadurch gekennzeichnet,**
**dass** eine Gesamtmenge von an die Zerstäuber (A, B, C, D) abgegebener Dekontaminationsflüssigkeit bestimmt wird durch Differenzbildung aus dem Gewicht der Dekontaminations-flüssigkeitsmenge im Dekontaminationsflüssigkeitsvorratsbehälter (36) bei mit Dekontaminationsflüssigkeit gefüllter Ringleitung (32) vor dem Dekontaminationszyklus und dem Gewicht der Dekontaminationsflüssigkeitsmenge im Dekontaminations-flüssigkeitsvorratsbehälter (36) bei mit Dekontaminationsflüssigkeit gefüllter Ringleitung (32) nach dem Dekontaminationszyklus und dass die als Zweistoffdüsen ausgebildeten Zerstäuber (A, B, C, D) gasleitend mit einer Gasdruckquelle, insbesondere einer Druckluftquelle (9) verbunden sind und ein Trägergasstrom für das zu erzeugende Dekontaminationsmittelaerosols erzeugen.

12. Dekontaminationsverfahren nach Anspruch 11,
**dadurch gekennzeichnet,**
**dass** die Ringleitung (32) vollständig mit Dekontaminationsflüssigkeit, insbesondere aus dem Dekontaminationsflüssigkeitsvorratsbehälter (36), bevorzugt mittels der Pumpe (35), gefüllt wird, bevor Dekontaminationsflüssigkeit in einem Dekontaminationszyklus durch entsprechende Ansteuerung der Ventilmittel (27) aus der Ringleitung (32) zu den Zerstäubern (A, B, C, D) strömt.

## Claims

1. A decontamination assembly, in particular for pharmaceutical applications, comprising at least one chamber (4) to be decontaminated, in particular an isolator chamber, preferably a manipulator chamber and/or a plenum chamber being separated from the manipulator chamber by a membrane, comprising a decontamination liquid storage tank (36) by which several atomizers (A, B, C. D) disposed in the chamber (4) to be decontaminated and preferably configured as two-component nozzles for producing a decontaminant aerosol can be supplied with decontamination liquid, preferably hydrogen peroxide, using valve means (27) which can be controlled by means of electronic control means, the atomizers (A, B, C, D) being in fluid communication with a ring conduit (32) having an inlet (33) and a separate outlet (34) by means of the valve means (27), decontamination liquid from the decontamination liquid storage tank (36) being conveyable into the inlet of said ring conduit by means of a pump (35) and decontamination liquid flowing out of the outlet of said ring conduit back into the decontamination liquid storage tank (36), and that pressure adjustment means (37) for keeping the pressure in the ring conduit (32) at a predefined liquid pressure range are assigned to the ring conduit (32),
**characterized in that**
measuring means (40) for determining a total volume of decontamination liquid dispensed to the atomizers (A, B, C, D) during a decontamination cycle are provided, in particular comprising weighing means (41) for determining a decontamination liquid volume in the decontamination liquid storage tank (36) and that the atomizers (A, B, C, D) which are configured as two-component nozzles are connected to a gas pressure source, in particular a compressed air source (9), in a gas-conducting manner to generate a carrier gas flow for the decontaminant aerosol to be produced.

2. The decontamination assembly according to claim 1,
**characterized in that**
the measuring means (40) are configured to determine the total volume of the decontamination liquid dispensed to the atomizers by defining the difference between the weight of the decontamination liquid volume in the decontamination liquid storage tank (36) when the ring conduit (32) is filled with decontamination liquid before the decontamination cycle and the weight of the decontamination liquid volume in the decontamination liquid storage tank (36) when the ring conduit (32) is filled with decontamination liquid after the decontamination cycle.

3. The decontamination assembly according to any one of the preceding claims,
**characterized in that**
the pressure adjustment means (37) comprise a non-return valve (38) disposed, in particular, in the ring conduit (32), the valve element being configured to open the non-return valve (38) towards the decontamination liquid storage tank (36) against the spring force of a return spring.

4. The decontamination assembly according to any one of the preceding claims,
**characterized in that**
the pressure adjustment means (37) are configured and tuned to the pump (35) in such a manner that the decontamination liquid flows through the outlet (34) back into the decontamination liquid storage tank (36) when the valve means (27) are opened.

5. The decontamination assembly according to any one of the preceding claims,
**characterized in that**
the valve means (27) comprise switch valves (28, 29, 30, 31), in particular 2-/2-way valves, which can be controlled in a cyclic manner, preferably individual atomizers (A, B, C, D), in particular all atomizers (A, B, C, D) being assigned such a switch valve (28, 29, 30, 31) in order to supply the respective atomizer (A, B, C, D) with decontamination liquid from the ring conduit (32) and/or that at least one group of atomizers (A, B, C, D), preferably several groups of atomizers (A, B, C, D) is/are assigned such a switch valve (28, 29, 30, 31).

6. The decontamination assembly according to claim 5,
**characterized in that**
the control means are configured to control the switch valves (28, 29, 30, 31) in such a manner that several, in particular all switch valves (28, 29, 30, 31) or groups of switch valves are simultaneously opened and/or are operated in a cycled manner and/or in such a manner that several switch valves (28, 29, 30, 31) or groups of switch valves are subsequently opened and/or are operated in a cycled manner and/or in such a manner that at least one of the switch valves (28, 29, 30, 31) or at least one of the groups of switch valves is opened for a longer period of time and/or operated in a cycled manner for a longer period of time during a decontamination cycle than at least one other switch valve (28, 29, 30, 31) or one other group of switch valves.

7. The decontamination assembly according to claim 5 or 6,
**characterized in that**
the control means are configured to control the switch valves (28, 29, 30, 31) in such a manner that a given total volume of decontamination liquid to be atomized can be dosed by controlling an in particular clocked total opening time of the switch valves (28, 29, 30, 31) and/or in such a manner that a given volume of decontamination liquid to be dosed can be dosed at one of the atomizers (A, B, C, D) or a group of atomizers (A, B, C, D) by controlling an in particular clocked opening time of the corresponding switch valve (28, 29, 30, 31).

8. The decontamination assembly according to any one of the preceding claims,
**characterized in that**
several atomizers (A, B, C, D) which are connected to the ring conduit (32), preferably the majority of the atomizers (A, B, C, D) which are connected to the ring conduit (32), preferably all atomizers (A, B, C, D) which are connected to the ring conduit (32) are equidistantly spaced apart from the ring conduit (32).

9. The decontamination assembly according to any one of the preceding claims,
**characterized in that**
the ring conduit (32) is assigned a connector valve (42) behind the pump (35) in order to establish and close a bypass between an area of the ring conduit (32) in front of and behind the atomizers (A, B, C, D) and/or that the pressure adjustment means (37) can be bypassed by means of a bypass valve (39) and/or that the ring conduit (32) is assigned a compressed gas connection, in particular a compressed air connection (43), for emptying the ring conduit (32).

10. A system comprising an isolator (2), preferably for pharmaceutical applications, comprising a chamber (4) to be decontaminated, in particular a manipulator chamber and/or a plenum chamber being separated from the manipulator chamber by a membrane for laminarizing an air flow of a circulation fan, and comprising a decontamination assembly (3) according to any one of the preceding claims.

11. A decontamination method, a chamber (4) to be decontaminated, in particular an isolator chamber, preferably a manipulator chamber and/or plenum chamber being separated from the manipulator chamber by a membrane, being decontaminated by producing a decontaminant aerosol from a decontamination liquid, in particular hydrogen peroxide, by means of atomizers (A, B, C, D) which are disposed in the chamber (4) to be decontaminated and which are preferably configured as two-component nozzles supplied with compressed air, said atomizers being supplied with the decontamination liquid from a decontamination liquid storage tank (36) via valve means (27), the atomizers (A, B, C, D) being in fluid communication with a ring conduit (32) having an inlet (33) and a separate outlet (34), decontamination liquid from the decontamination liquid storage tank (36) being conveyed into the inlet of said ring conduit by means of a pump (35) and decontamination liquid flowing out of the outlet of said ring conduit back into the decontamination liquid storage tank (36), and that a liquid pressure of the decontamination liquid in the ring conduit (32) is kept at a predefined pressure range by means of pressure adjustment means (37),
**characterized in that**
a total volume of the decontamination liquid dispensed to the atomizers (A, B, C, D) is determined by defining the difference between the weight of the decontamination liquid volume in the decontamination liquid storage tank (36) when the ring conduit (32) is filled with decontamination liquid before the decontamination cycle and the weight of the decontamination liquid volume in the decontamination liquid storage tank (36) when the ring conduit (32) is filled with decontamination liquid after the decontamination cycle and that the atomizers (A, B, C, D) which are configured as two-component nozzles are connected to a gas pressure source, in particular a compressed air source (9), in a gas-conducting manner and generate a carrier gas flow for the decontaminant aerosol to be produced

12. The decontamination method according to claim 11,
**characterized in that**
the ring conduit (32) is filled completely with the decontamination liquid, in particular from the decontamination liquid storage tank (36), preferably by means of a pump (35) before the decontamination liquid flows from the ring conduit (32) to the atomizers (A, B, C, D) in a decontamination cycle by a corresponding control of the valve means (27).

## Revendications

1. Système de décontamination, notamment pour des applications pharmaceutiques, comprenant au moins une chambre (4) à décontaminer, notamment une chambre d'isolateur, de préférence une chambre de manipulation et/ou une chambre de plénum qui est séparée de la chambre de manipulation par une membrane, comprenant un réservoir de décontaminant liquide (36) par lequel plusieurs atomiseurs (A, B, C, D) disposés dans la chambre (4) à décontaminer et configurés de préférence comme une buse à deux substances pour produire un aérosol de décontaminant, de préférence peroxyde d'hydrogène, sont alimentés en utilisant des moyens de soupape (27) qui peuvent être contrôlés par des moyens de commande électroniques, les atomiseurs (A, B, C, D) étant disposés en communication fluide avec une conduite annulaire (32), qui a une entrée (33) et une sortie (34) séparée, par les moyens de soupape (27), le décontaminant liquide du réservoir de décontaminant liquide (36) pouvant être convoyé à l'entrée par une pompe (35) et le décontaminant liquide pouvant revenir de la sortie dans le réservoir de décontaminant liquide (36), et que des moyens de régulation de pression (37) destinés à maintenir la pression du liquide dans la conduite annulaire (32) à une plage de pression du liquide définie sont assignés à la conduite annulaire (32),
**caractérisé en ce que**
des moyens de mesure (40) destinés à déterminer un volume total du décontaminant liquide distribué aux atomiseurs (A, B, C, D) pendant un cycle de décontamination sont prévus, notamment comprenant des moyens de pesage (41) destinés à déterminer le volume du décontaminant liquide dans le réservoir de décontaminant liquide (36) et que les atomiseurs (A, B, C, D), qui sont configurés comme des buses à deux substances, sont reliés à une source de pression de gaz en transmettant du gaz, notamment une source d'air comprimé (9), pour générer un flux de gaz porteur pour l'aérosol de décontaminant à produire.

2. Système de décontamination selon la revendication 1,
**caractérisé en ce que**
les moyens de mesure (40) sont configurés pour déterminer le volume total du décontaminant liquide distribué aux atomiseurs par calculer la différence entre le poids du volume de décontaminant liquide dans le réservoir de décontaminant liquide (36) quand la conduite annulaire (32) est remplie de décontaminant liquide avant le cycle de décontamination et le poids du volume de décontaminant liquide dans le réservoir de décontaminant liquide (36) quand la conduite annulaire (32) est remplie de décontaminant liquide après le cycle de décontamination.

3. Système de décontamination selon l'une quelconque des revendications précédentes,
**caractérisé en ce que**
les moyens de régulation de pression (37) comprennent un clapet de non-retour (38) disposé notamment dans la conduite annulaire (32), l'élément de soupape étant configuré pour ouvrir le clapet de non-retour (38) vers le réservoir de décontaminant liquide (36) dans le sens contraire à la force de ressort d'un ressort de rappel.

4. Système de décontamination selon l'une quelconque des revendications précédentes,
**caractérisé en ce que**
les moyens de régulation de pression (37) sont configurés et adaptés à la pompe (35) de telle manière que le décontaminant liquide retourne dans le réservoir de décontaminant liquide (36) par la sortie (34) quand les moyens de soupape (27) sont ouverts.

5. Système de décontamination selon l'une quelconque des revendications précédentes,
**caractérisé en ce que**
les moyens de soupape (27) comprennent des soupapes de commutation (28, 29, 30, 31), notamment des soupapes à 2/2 voies, qui peuvent être contrôlées notamment de manière cadencée, ladite soupape de commutation (28, 29, 30, 31) étant assignée, de préférence, aux atomiseurs (A, B, C, D) individuels, notamment aux tous les atomiseurs (A, B, C, D) afin d'alimenter ledit atomiseur (A, B, C, D) en décontaminant liquide de la conduite annulaire (32) et/ou que ladite soupape de commutation (28, 29, 30, 31) est assignée à au moins un groupe d'atomiseurs (A, B, C, D), de préférence aux plusieurs groupes d'atomiseurs (A, B, C, D).

6. Système de décontamination selon la revendication 5,
**caractérisé en ce que**
les moyens de commande sont configurés pour contrôler les soupapes de commutation (28, 29, 30, 31) de telle manière que plusieurs, notamment tous les soupapes de commutation (28, 29, 30, 31) ou groupes de soupapes de commutation sont ouverts et/ou exploités de manière cadencée simultanément et/ou de telle manière que plusieurs soupapes de commutation (28, 29, 30, 31) ou groupes de soupapes de commutation sont ouverts et/ou exploités de manière cadencée successivement et/ou de telle manière qu'au moins une soupape de commutation (28, 29, 30, 31) ou au moins un groupe de soupapes de commutation est ouvert/e pendant une période de temps plus longue et/ou exploité de manière cadencée pendant une période de temps plus longue qu'au moins une autre soupape de commutation (28, 29, 30, 31) ou un autre groupe de soupapes de commutation.

7. Système de décontamination selon la revendication 5 ou la revendication 6,
**caractérisé en ce que**
les moyens de commande sont configurés pour contrôler les soupapes de commutation (28, 29, 30, 31) de telle manière qu'un volume total défini du décontaminant liquide à atomiser peut être dosé par une commande du temps total d'ouverture, notamment de manière cadencée, des soupapes de commutation (28, 29, 30, 31) et/ou de telle manière qu'un volume défini du décontaminant liquide à doser peut être dosé à un atomiseur (A, B, C, D) ou un groupe d'atomiseurs (A, B, C, D) par une commande du temps d'ouverture, notamment de manière cadencée, de la soupape (28, 29, 30, 31) correspondante.

8. Système de décontamination selon l'une quelconque des revendications précédentes,
**caractérisé en ce que**
plusieurs atomiseurs (A, B, C, D) qui sont raccordés à la conduite annulaire (32), de préférence la plupart des atomiseurs (A, B, C, D) qui sont raccordés à la conduite annulaire (32), de préférence tous les atomiseurs (A, B, C, D) qui sont raccordés à la conduite annulaire (32) sont espacés de façon équidistante de la conduite annulaire (32).

9. Système de décontamination selon l'une quelconque des revendications précédentes,
**caractérisé en ce**
**qu'**une soupape de liaison (42) destinée à établir et à fermer une dérivation entre une partie de la conduite annulaire (32) devant et derrière les atomiseurs (A, B, C, D) est assignée à la conduite annulaire (32) à l'arrière de la pompe (35) et/ou que les moyens de régulation de pression peuvent être bypassés par une soupape de dérivation (39) et/ou qu'un raccord de gaz comprimé, notamment un raccord d'air comprimé (43) destiné à vider la conduite annulaire (32) est assignée à la conduite annulaire (32).

10. Système comprenant un isolateur (2), de préférence pour des applications pharmaceutiques, comprenant une chambre (4) à décontaminer, notamment une chambre de manipulation et/ou une chambre de plénum qui est séparée de la chambre de manipulation par une membrane afin de rendre laminaire le flux d'air d'un ventilateur de circulation, et comprenant un système de décontamination (3) selon l'une quelconque des revendications précédentes.

11. Procédé de décontamination, une chambre (4) à décontaminer, notamment une chambre d'isolateur, de préférence une chambre de manipulation et/ou une chambre de plénum qui est séparée de la chambre de manipulation par une membrane, étant décontaminée en produisant un aérosol de décontaminant, de préférence peroxyde d'hydrogène, d'un décontaminant liquide au moyen des atomiseurs (A, B, C, D) disposés dans la chambre (4) à décontaminer et configurés de préférence comme une buse à deux substances qui sont alimentés en décontaminant liquide d'un réservoir de décontaminant liquide (36) par les moyens de soupape (27), les atomiseurs (A, B, C, D) étant disposés en communication fluide avec une conduite annulaire (32) ayant une entrée (33) et une sortie (34) séparée par les moyens de soupape (27), le décontaminant liquide du réservoir de décontaminant liquide (36) étant convoyé à l'entrée par une pompe (35) et le décontaminant liquide pouvant revenant de la sortie dans le réservoir de décontaminant liquide (36), notamment pendant la distribution de décontaminant liquide par les atomiseurs (A, B, C, D), et qu'une pression du liquide de décontaminant liquide dans la conduite annulaire (32) est maintenue à une plage de pression du liquide définie par des moyens de régulation de pression (37),
**caractérisé en ce que**
le volume total du décontaminant liquide distribué aux atomiseurs (A, B, C, D) est déterminé par calculer la différence entre le poids du volume de décontaminant liquide dans le réservoir de décontaminant liquide (36) quand la conduite annulaire (32) est remplie de liquide avant le cycle de décontamination et le poids du volume de décontaminant liquide dans le réservoir de décontaminant liquide (36) quand la conduite annulaire (32) est remplie de liquide après le cycle de décontamination et que les atomiseurs (A, B, C, D), qui sont configurés comme des buses à deux substances, sont reliés à une source de pression de gaz en transmettant du gaz, notamment une source d'air comprimé (9) et que les atomiseurs génèrent un flux de gaz porteur pour l'aérosol de décontaminant à produire.

12. Procédé de décontamination selon la revendication 11,
**caractérisé en ce que**
la conduite annulaire (32) est entièrement remplie de décontaminant liquide, notamment du réservoir de décontaminant liquide (36), de préférence au moyen de la pompe (35) avant que la décontaminant liquide passe de la conduite annulaire (32) aux atomiseurs (A, B, C, D) dans un cycle de décontamination par un contrôle correspondant des moyens de soupape (27).
